# FASCICULE DE BREVET EUROPEEN

(11) **EP 1 670 982 B1**
(45) Date de publication et mention de la délivrance du brevet: **22.04.2009**
(21) Numéro de dépôt: 04787488.8
(22) Date de dépôt: 30.09.2004
(51) Int. Cl.: D04H 13/00

(54) **ARTICLE POUR LES SOINS DE LA PEAU**
HAUTPFLEGEERZEUGNIS
SKIN CARE ARTICLE

(30) Priorité: 02.10.2003 FR 0311560
(43) Date de publication de la demande: 21.06.2006
(73) Titulaire: Georgia-Pacific France, 68320 Kunheim (FR)
(72) Inventeur: LOUIS DIT PICARD, Bernard, F-27370 Amfreville-la-Campagne (FR); GREGOIRE, Philippe, F-27700 Les Andelys (FR)
(74) Mandataire: Cortier, Sophie
(86) Numéro de dépôt international: PCT/FR2004/002475
(87) Numéro de publication internationale: WO 2005/033392

(56) Documents cités:
- EP-A- 0 405 043
- EP-A- 0 851 052
- EP-A- 1 167 605
- WO-A-00/76384
- WO-A-94/02674
- FR-A- 2 052 089
- US-A- 5 437 372

## Description

La présente invention porte sur un article, constitué essentiellement de fibres, destiné aux soins cosmétiques de la peau et ayant en particulier la propriété d'avoir un effet gommant.

Dans le cadre des soins de la peau, dans le domaine des produits cosmétiques notamment, on connaît le traitement d'exfoliation ou de gommage. Il s'agit d'une opération qui permet, par une action mécanique de frottement, l'élimination des impuretés, incrustées et accumulées à la surface de l'épiderme, et des cellules mortes. Le frottement permet d'affiner le grain de la peau, de purifier l'épiderme et d'éclaircir le teint. On utilise habituellement des fibres naturelles. On connaît par exemple le pain de luffa ou courge fibreuse, le gant de crin, de chanvre ou même de sisal. Cependant, ces fibres grossières sous cette forme ont pour effet un gommage fort qui ne peut être réalisé que de temps en temps, de façon hebdomadaire ou encore mensuelle. On connaît aussi des préparations exfoliantes avec des éléments naturels, organiques ou de type végétal comme les akènes de fraise, noyaux d'abricot concassés, silice organique de bambou, cellulose de courge, ou bien encore des éléments de type minéral comme des billes de silice ou bien encore des éléments artificiels et / ou synthétiques. L'ensemble de ces derniers comprend tous les polymères tels que : polyéthylène, polyamide 6.6, polypropylène, EVA.

On propose actuellement des tampons en forme de disque constitué d'un substrat absorbant en matière spongieuse ou fibreuse, comportant en surface des éléments ayant un effet gommant. Par exemple la demande de brevet US 2002/0087167 décrit un tel article de nettoyage de la peau avec une feuille abrasive en surface. Cette feuille en tissu de coton ou de lin ou en un autre matériau équivalent supporte de fins grains de sable ou autre matériau abrasif, dispersés en surface. Les grains sont choisis pour leur utilisation en micro-dermo-abrasion. Ce sont des microbilles qui permettent des gommages plus doux. On ajoute aussi des sphères cassables larguant des principes actifs. Cependant dans le cas d'un substrat fibreux, l'incorporation d'une matière sous 1a forme de particules et la liaison de ces dernières aux fibres sont des facteurs qui rendent 1e procédé de fabrication de l'article plus complexe et limitent les cadences de production. Il faut par exemple prêter une attention particulière au traitement des chutes produites au cours de la découpe de la nappe en tampons individuels. Leur recyclage pose problème car on est susceptible de polluer les fibres avec les particules abrasives.

L'invention a pour objet la réalisation d'un produit ayant à la fois un effet gommant et une certaine capacité d'absorption des liquides.

La présente invention vise aussi la réalisation d'un produit destiné aux soins de la peau et ayant un effet gommant mettant en oeuvre seulement des techniques textiles tout en permettant le recyclage des chutes.

L'invention a en particulier pour objet un produit que l'on peut fabriquer sur une installation existante de fabrication de produits en coton ou mélange de fibres se substituant au coton sans nécessiter de modifications substantielles.

L'invention a également pour objet un produit pouvant être composé uniquement de fibres cellulosiques naturelles.

On parvient à réaliser ces objectifs conformément à l'invention avec un article pour le soin notamment cosmétique de la peau avec un effet de gommage par frottement comprenant au moins une première et une seconde couches de matières fibreuses en particulier de fibres cellulosiques, caractérisé par le fait que la première couche forme un matelas absorbant et est composée essentiellement de fibres cellulosiques de faible micronaire notamment inférieur à 8, et la deuxième couche est constituée essentiellement de fibres à haut micronaire, en particulier de micronaire supérieur à 8 choisies parmi les matériaux suivants : lin, ramie, sisal, jute, chanvre, seuls ou en mélange.

En particulier, la deuxième couche comprend au moins 50%, de préférence 70% de fibres à haut micronaire, et au plus 50%, de préférence 30% d'autres fibres, naturelles, synthétiques ou artificielles.

La première couche comprend au moins 50%, en particulier au moins 70%, et plus précisément 100% desdites fibres cellulosiques,

De préférence, les couches sont liées entre elles. Avantageusement, elles sont liées selon une technique de liage hydraulique désignée aussi hydroliage. On obtient ainsi un produit présentant une résistance à la rupture comprise entre 10 et 60 N pour une éprouvette de 25 mm de large.

Grâce à l'invention, on réalise un produit présentant une partie absorbant les liquides par les fibres de coton hydrophile, éventuellement combinées à d'autres fibres telles que des fibres artificielles et/ou synthétiques, et une partie ayant un effet légèrement abrasif sur la peau que sont les fibres à haut micronaire. L'ensemble forme après liage des fibres un produit présentant de manière surprenante une parfaite cohésion.

La demanderesse est titulaire du brevet EP1189531. Il concerne un produit de coton hydrophile constitué à cent pour cent de fibres de coton et comprenant une première couche constituée de fibres fines présentant un indice micronaire faible formant une face douce et une seconde couche extérieure constituée de fibres présentant un indice micronaire plus élevé formant une face grattante. Les valeurs mentionnées sont un indice micronaire compris entre 2 et 5 µg/pouce pour la première couche, et un indice micronaire compris entre 4 et 10 µg/pouce pour la seconde couche. En fait, dans la pratique, l'indice micronaire de la seconde couche reste inférieur à 8,5 µg/pouce, le coton n'ayant qu'exceptionnellement un indice micronaire de valeur supérieure à 7,5. On précise qu'usuellement, quand on fait référence à l'indice micronaire, on ne mentionne pas l'unité µg/pouce. Le produit selon le brevet est destiné à un usage cosmétique pour maquiller et/ou démaquiller la peau.

La présente invention se distingue de cet art antérieur par le fait que le tampon n'est pas seulement destiné à un simple nettoyage de la peau comme on le pratique au moment du démaquillage. Si les caractéristiques du tampon selon cet art antérieur permettent d'enlever les pommades, fards, rouge à lèvre ou autres substances de maquillage, elles ne sont pas adaptées à une opération de gommage qui met en oeuvre les propriétés abrasives du substrat. On a constaté avec surprise que l'on pouvait conférer à un tampon fibreux, en plus de sa fonction démaquillante, une fonction de gommage de la peau par la simple substitution d'une partie des fibres de coton par des fibres à haut micronaire, plus grosses et plus rigides.

En outre, ce nouveau produit est obtenu sans avoir à modifier de manière substantielle l'équipement industriel de fabrication des tampons. Cela est particulièrement avantageux lorsqu'on souhaite fabriquer des produits efficaces, économiques et de grande diffusion.

On observe en outre que dans le cas de fibres de lin, l'indice micronaire élevé résulte de l'amas de fibres et non de la grosseur de la fibre individuelle. Comme on le présente plus loin le traitement de débouillissage et de blanchiment conventionnel, tel qu'appliqué au coton, ne conduit pas à une séparation suffisante des fibres pour les individualiser.

On décrit l'invention plus en détail ci-après en référence aux dessins en annexe dans lesquels :
- la figure 1 représente en coupe transversale, un exemple de réalisation d'un article de l'invention,
- la figure 2 représente un deuxième mode de réalisation de l'invention, et
- la figure 3 représente un troisième mode de réalisation de l'invention.

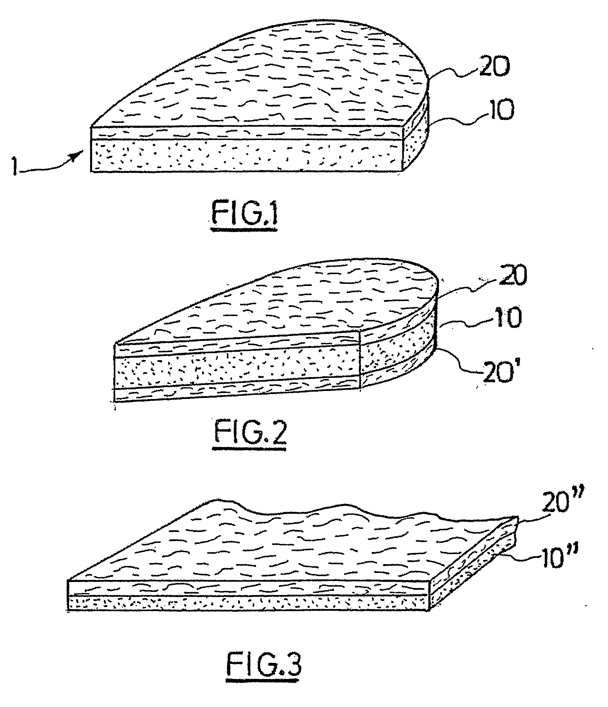

Comme on le voit sur la figure 1, l'article ou tampon 1 commercialisé pour un usage cosmétique avec une opération de gommage de la peau forme un disque ou un format au contour quelconque. Il peut être circulaire, ovale, polygonal ou de toute autre forme. Il est constitué d'une première couche de fibres 10 formant un matelas absorbant les liquides. Ce dernier est composé essentiellement de fibres de coton blanchi. Ces fibres peuvent être mélangées en partie à d'autres fibres utilisées communément dans le domaine des articles à usage cosmétique, telles que des fibres artificielles comme la viscose ou bien des fibres synthétiques, hydrophiles ou non. Selon la technique de découpe des tampons utilisée, on peut être amené à recycler une partie de la nappe initiale. Ces fibres recyclées sont alors incorporées aux fibres neuves constituant la première couche. La quantité de fibres qui ne sont pas de coton est de préférence limitée à 50% et plus précisément à 30% du total des fibres de la couche.

Le poids du matelas est compris entre 30 et 300 g/m². Il peut s'agir d'une nappe formée par voie aéraulique, par une machine de type Rando Webber qui dispose les fibres avec une certaine orientation inclinée par rapport à la direction de formation de la nappe. La couche est ainsi relativement épaisse. La nappe peut être associée à un ou plusieurs voiles de carde comme dans la nappe formée selon le procédé de blanchiment en continu et décrite dans le brevet de la demanderesse EP 0 681 621.

Cette couche peut aussi être fabriquée entièrement à partir d'une ou de plusieurs cardes et être ainsi constituée d'un ensemble de voiles de carde.

Grâce aux fibres de coton blanchi, hydrophile, le matelas absorbe très bien les produits utilisés dans le maquillage, le démaquillage ou autres soins de la peau.

La seconde couche 20 est composée de fibres à haut micronaire, supérieur à 8. De préférence, il s'agit de fibres naturelles, avantageusement choisies parmi le lin, le chanvre, le sisal, le jute ou la ramie, seul ou en mélange. Les fibres sont également blanchies. Le poids de la seconde couche est de préférence compris entre 15 et 120 g/m². Les fibres forment ensemble une nappe fine obtenue par une machine de nappage connue. Elles peuvent aussi être disposées en un ou plusieurs voiles de carde superposés. La couche 20 peut aussi être réalisée par voie aéraulique. De préférence, la seconde couche est constituée exclusivement de fibres à haut micronaire comme le lin, cependant, elle peut comprendre minoritairement d'autres fibres.

Dans le cas du lin, les fibres ont individuellement un diamètre compris entre 0,015 et 0,025 mm. Il est peu différent de celui des fibres de coton qui est compris entre 0,015 et 0,04 mm. Cependant, en raison des matières pectiques qui entre-collent les liasses de fibres, elles sont difficilement individualisables par les procédés de blanchiment du coton conventionnels. Elles forment des amas ou bûchettes. Le diamètre réel des amas apparaît plus grand, et ils sont plus rigides que les fibres elles-mêmes. Alors que l'indice micronaire du coton est compris généralement entre 2 et 8 selon l'origine de la fibre, celui des fibres de lin blanchi est supérieur à 8. On profite de cette propriété pour réaliser une couche à effet gommant. On élargit la réalisation de l'invention aux fibres naturelles dont le micronaire est supérieur à 8 après qu'elles aient été blanchies, dans les conditions usuelles de blanchiment du coton. Par exemple, on ne procède pas à un cardage supplémentaire après débouillissage et blanchiment. Les fibres naturelles visées sont le sisal, le chanvre, le jute ou la ramie.

On rappelle que l'indice micronaire représente la masse moyenne des fibres par unité de longueur, en µg/pouce, d'un échantillon de produit de matière fibreuse testée. Cet indice est directement lié à la section moyenne des fibres ou amas de fibres. Il définit la grosseur des fibres ou amas de fibres et se mesure au moyen d'un appareil de mesure tel que le SHEFFIELD Micronaire suivant une méthode définie, par exemple la méthode ISO 2403 ou NF G 07-073.

Sa mesure se base sur la perméabilité à l'air d'une masse de fibres dans certaines conditions spécifiques, selon une échelle arbitraire appelée échelle micronaire. On mesure la perméabilité à l'air d'un échantillon donné, prélevé dans une couche extérieure du produit selon l'invention (constituant l'éprouvette d'essai), par lecture de la résistance au passage du flux d'air sur l'appareil à flux d'air, sur une échelle graduée en fonction de variations du débit d'air ou de différences de pression. Cette échelle a été préalablement étalonnée avec une série de cotons de référence.

L'appareillage comprend une balance pour la mesure de la masse de l'éprouvette, un appareil à flux d'air permettant la mesure de l'indice micronaire, consistant en un cylindre de compression perforé contenant l'éprouvette d'essai et des dispositifs nécessaires pour la mesure de la perméabilité à l'air de l'éprouvette, tels qu'une pompe à air, des moyens de réglage du flux d'air ou de la pression passant à travers l'éprouvette dans le cylindre de compression, un manomètre pour lire la différence de pression requise et un débit-mètre pour la lecture du débit d'air au travers de l'éprouvette, et si nécessaire une échelle graduée en valeurs d'indice micronaire ou une table fournissant la conversion des lectures en indice micronaire. L'échantillonnage et le prélèvement des éprouvettes se font par exemple conformément aux normes NF G 07-050 et NF G 07-062.

On détermine ensuite la masse de l'éprouvette. On introduit uniformément et par petites quantités l'éprouvette dans le cylindre de compression et l'on met en place le piston de compression des fibres, que l'on verrouille ensuite. On ouvre l'arrivée d'air à la pression ou au débit approprié et on note la différence de pression sur l'échelle de l'instrument.

Pour des appareils dont l'échelle est graduée en valeurs d'indice micronaire, on calcule la moyenne des lectures obtenues pour l'ensemble des éprouvettes prélevées sur un même échantillon. Pour des appareils dont l'échelle est graduée en d'autres unités qu'en valeurs d'indice micronaire, on convertit les lectures directes en indices micronaires à partir de la courbe de conversion.

L'indice micronaire représentant la masse moyenne des fibres à l'unité de longueur, s'exprime en µg/pouce.

Avantageusement les couches 10 et 20 sont liées entre elles. Elles peuvent avoir subi un aiguilletage mécanique ou hydraulique. De préférence, on procède par liage au moyen de jets d'eau. L'énergie appliquée par les jets sur la face comprenant la seconde couche est comprise entre 0,7x10⁻³ et 10x10⁻³ kWh/m², selon l'état des fibres du matelas pendant le traitement.

On a constaté avec surprise que des tampons présentant cette structure avaient une excellente tenue et une résistance à la rupture supérieure à celle que l'on attendait. Cette dernière propriété est importante dans une application où l'on est amené à frotter le tampon sur la peau. Il remplit mieux sa fonction de nettoyage de la surface et libère moins de fibres par peluchage.

Parmi les fibres à haut micronaire, on sait par exemple que le lin bénéficie d'une ténacité à sec supérieure à celle du coton. Elle est comprise entre 40 et 60 g/tex contre un intervalle de 25 à 40 g/tex pour le coton. De plus, sa ténacité au mouillé, condition majoritaire de son utilisation, est supérieure de 50 à 80% par rapport à celle à sec.

Ainsi on a comparé à sec un tampon entièrement en coton de 250 g/m² à un tampon constitué d'un matelas de coton de même grammage auquel on a associé une couche de voiles de carde de fibres de lin de 40 g/m².

Le premier présentait une résistance à la rupture sens marche de 17 N, alors que la résistance du second selon l'invention atteignait 40 N. On est au-delà de la valeur attendue de la résistance d'un tampon entièrement en coton qui serait de 30 N compte-tenu de l'augmentation du grammage.

La figure 2 montre un autre mode de réalisation dans lequel la première couche 10 absorbante formée essentiellement de fibres de coton, est comprise entre deux couches 20 et 20' de fibres à haut micronaire, telles que le lin. La première couche a un grammage compris entre 30 et 300 g/m² et chacune des deux couches de fibres de lin a un grammage compris entre 15 et 120 g/m² comme dans le premier mode de réalisation.

La figure 3 montre un autre mode de réalisation sous la forme d'un non-tissé léger, de grammage compris entre 45 et 100 g/m² avec une couche 10" de coton et une couche 20" de fibres à haut micronaire. Les couches sont sensiblement de même épaisseur, faible. Ce non-tissé, par son application à un usage cosmétique, peut être imprégné ou non d'une solution traitante ou de démaquillage. Ce type de produit est connu sous le nom dans le domaine cosmétique de lingette démaquillante ou exfoliante.

Pour fabriquer un tampon ou une lingette conforme à l'invention, on prépare par des moyens de nappage textiles connus en soi, une nappe de fibres dont le grammage est de préférence compris entre 30 et 300 g/m². La nappe comprend avantageusement au moins 70% de fibres de coton blanchi. Les fibres le plus souvent utilisées avec le coton sont la viscose ou le polyester. On dépose sur cette première couche de fibres, une deuxième couche constituée d'un ou de plusieurs voiles de carde de fibres naturelles blanchies à haut micronaire, par exemple de lin blanchi. Le grammage de cette deuxième couche est compris entre 15 et 120 g/m². Le cas échéant, on dépose une troisième couche de fibres naturelles blanchies à haut micronaire, sur la face opposée de la nappe de coton.

L'ensemble est guidé à travers un poste de liage des couches entre elles et de consolidation. Avantageusement, il s'agit d'un liage au moyen de jets d'eau. Un procédé connu d'hydroliage consiste à traiter la structure fibreuse à l'aide de jets d'eau à haute pression, dans le but d'emmêler tout ou partie des fibres et de modifier certaines de ses caractéristiques. On cherche en particulier par ce procédé à en modifier la résistance mécanique et le peluchage. Le matelas de fibres est supporté par une toile poreuse qui se déplace dans une direction perpendiculaire par rapport aux jets d'eau. Ces derniers sont produits par un appareil comportant une ou plusieurs rangées d'injecteurs disposés en travers par rapport à la direction de déplacement de la nappe de fibres. On réalise habituellement les injecteurs en usinant des perforations calibrées de forme circulaire, toutes de même diamètre et de profil adapté dans une lame métallique. Cette lame recouvre un canal de distribution alimenté par des pompes fournissant l'eau à une pression élevée.

Pour l'application ici décrite, les jets d'eau sont réglés pour apporter une énergie de 6,6x10⁻³ kWh/m² pour la face lin et 0,9x10⁻³ kWh/m² sur l'autre face. Les fibres s'entremêlent sous l'action des jets. Les perforations ont couramment un diamètre compris entre 80 µm et 200µm et sont espacées le long de la lame. L'espacement est compris entre 0,5 à 8 mm. On trouve dans le commerce des lames métalliques pourvues d'une à trois rangées de perforations.

La toile poreuse, sur laquelle le matelas de fibres est étendu, est entraînée le long d'une table plane ou bien sur un cylindre mis en rotation. La toile permet à l'eau de traverser la nappe fibreuse, et un moyen d'aspiration ménagé sous la toile en assure l'évacuation.

A partir d'un certain grammage ou d'épaisseur de la nappe, ce dispositif a pour résultat immédiatement visible la réalisation d'un relief formé de lignes continues, rectilignes, parallèles entre elles. Ces lignes sont alignées dans le sens de défilement de la nappe par rapport aux injecteurs.

La nappe peut être consolidée par des moyens connus autres qui sont par exemple le calandrage, l'aiguilletage mécanique ou bien le traitement thermique en combinaison avec l'incorporation de poudres ou de fibres fusibles. On sèche la nappe le cas échéant. On découpe ensuite la nappe en tampons ou formats individuels. Les moyens de découpe peuvent par exemple être des emporte-pièce ou des couteaux montés sur des cylindres rotatifs.

Selon un autre mode de fabrication, on prépare une première couche de fibres de coton d'une part et au moins une deuxième couche de fibres naturelles choisies parmi le lin, le sisal, le chanvre, le jute et la ramie sous la forme de voiles de carde d'autre part. Les fibres sont écrues ; elles n'ont pas encore subi de traitement chimique de débouillissage et blanchiment. La nappe formée des différentes couches est traitée par des liquides de débouillissage et de blanchiment, soit en continu comme cela est décrit dans le brevet déposé par la demanderesse, EP 0 524 268, soit en discontinu selon les techniques traditionnelles ou bien tel que décrit dans les brevets EP 0 735 175 et FR 2 552 120.

L'opération de liage, avantageusement par jets d'eau, est effectuée antérieurement ou postérieurement au traitement chimique.

Les techniques, décrites dans les brevets mentionnés ci-dessus, assurent une excellente cohésion des fibres à l'intérieur de chaque couche et entre les couches, contribuant à la réalisation d'un produit remarquablement homogène quant à son aspect.

## Revendications

1. Article pour le soin de la peau avec un effet de gommage par frottement comprenant au moins une première et une seconde couches extérieures de matières fibreuses, **caractérisé par le fait que** la première couche (10, 10") forme un matelas absorbant et est composée essentiellement de fibres cellulosiques de faible micronaire, en particulier inférieur à 8, et la deuxième couche (20, 20', 20") est constituée essentiellement de fibres à haut micronaire, en particulier de micronaire supérieur à 8, choisies parmi les matériaux suivants : lin, ramie, sisal, jute, chanvre, seuls ou en mélange.

2. Article selon la revendication précédente dont la deuxième couche (20, 20', 20") comprend au moins 50%, de préférence 70% de fibres à haut micronaire, et au plus 50% de préférence 30% d'autres fibres, naturelles, synthétiques ou artificielles.

3. Article selon l'une des revendications 1 et 2, dont la première couche comprend au moins 50%, en particulier au moins 70%, et plus précisément 100% desdites fibres cellulosiques,

4. Article selon la revendication 3 dont les dites fibres cellulosiques de faible micronaire sont des fibres de coton.

5. Article selon l'une des revendications précédentes, dont le grammage de la première couche (10) est compris entre 30 et 300 g/m².

6. Article selon la revendication 3 dont la première couche comprend jusqu'à 50%, et en particulier jusqu'à 30% de fibres synthétiques et/ou artificielles.

7. Article selon l'une des revendications 3 à 6 dont la première couche (10) comprend une nappe de fibres formée par voie aéraulique ou par cardage.

8. Article selon l'une des revendications précédentes dont la première couche (10) comprend un ou plusieurs voiles de carde superposés.

9. Article selon la revendication 1 ou 2 dont le grammage de la seconde couche (20, 20' 20") est compris entre 15 et 120 g/m².

10. Article selon l'une des revendications précédentes comprenant une troisième couche (20') constituée essentiellement de fibres à haut micronaire, la première couche (10) étant disposée entre la deuxième et la troisième couche.

11. Article selon l'une des revendications précédentes dont les fibres cellulosiques de la seconde couche sont des fibres de lin.

12. Article selon l'une des revendications précédentes dont la seconde couche (20, 20") et/ou la troisième couche (20', 20") le cas échéant sont formées de voiles de carde.

13. Article selon l'une des revendications précédentes dont la seconde couche ou la troisième couche le cas échéant comprend une nappe formée par voie aéraulique.

14. Article selon l'une des revendications précédentes dont les couches sont liées entre elles.

15. Article selon la revendication précédente dont les couches sont liées entre elles par hydroliage.

16. Article selon l'une des revendications 14 et 15 dont la résistance à la rupture est comprise entre 10 et 60 N pour une éprouvette de 25 mm de large.

17. Article selon la revendication précédente se présentant sous la forme d'une lingette de grammage compris entre 45 et 100 g/m².

18. Procédé de fabrication d'un article selon l'une des revendications 15 et 16 dont l'énergie d'hydroliage appliquée à la face comportant la deuxième couche est comprise entre 0,7x10⁻³ kWh/m² et 10x10⁻³ kWh/m².

19. Procédé selon la revendication précédente dont les couches de fibres sont formées à partir de fibres naturelles écrues et sont hydroliées avant de subir un traitement chimique de débouillissage et blanchiment.

20. Procédé selon la revendication 18 dont les couches de fibres sont formées à partir de fibres naturelles écrues et sont débouillies et blanchies avant d'être hydroliées.

## Claims

1. An article for skin care with an exfoliating effect produced by rubbing and comprising at least one first and one second layer of fibrous materials, **characterized by** the fact that the first layer (10, 10") forms an absorbent mat and is comprised essentially of cellulose fibres with a low micronaire, in particular a micronaire lower than 8, while the second layer (20, 20', 20") is comprised essentially of cellulose fibres with a high micronaire, in particular a micronaire higher than 8, said fibres being selected from among the following materials: flax, ramie, sisal, jute, hemp, either individually or as a blend.

2. The article as specified in the preceding claim whose second layer (20, 20', 20") comprises at least 50%, preferably 70% of high-micronaire fibres, and at the most 50%, preferably 30%, of other fibres, whether natural, synthetic or artificial.

3. The article as specified in either of Claims 1 and 2 whose first layer comprises at least 50%, in particular at least 70%, and more specifically 100%, of the said cellulose fibres.

4. The article according to Claim 3 whose low micronaire cellulose fibres are cotton.

5. The article according to any of the preceding claims whose grammage of the first layer (10) ranges from 30 to 300 g/m².

6. The article according to Claim 3 whose first layer comprises up to 50% and in particular up to 30% of synthetic and/or artificial fibres.

7. The article according to any of the Claims 3 to 6 whose first layer (10) comprises a sheet of fibres formed by an air-laid means or by carding.

8. The article according any of the preceding claims whose first layer (10) comprises one or several superimposed carded webs.

9. The article according to Claim 1 or 2 whose grammage of the second layer (20, 20', 20") is between 15 and 120 g/m³.

10. The article according to one of the preceding claims comprising a third layer (20') made essentially of fibres with a high micronaire, the first layer (10) being arranged between the second and the third layer.

11. The article according to one of the preceding claims whose cellulose fibres of the second layer are flax.

12. The article according to one of the preceding claims whose second layer (20, 20") and/or the third layer (20', 20") are, in particular, formed by carded webs.

13. The article according to one of the preceding claims whose second layer or third layer, in particular, comprises a sheet formed by means of an air-laid method.

14. The article according to one of the preceding claims whose layers are bonded together.

15. The article according to the preceding claim whose layers are bonded together by means of hydroentangling.

16. The article according to one of Claims 14 and 15 whose breaking strength is between 10 and 60 N for a specimen 25 mm wide.

17. The article according to the preceding claim presenting in the form of a wipe with a grammage between 45 and 100 g/m².

18. A process for manufacturing an article according to either of Claims 15 and 16 whose hydroentangling energy applied to the facing comprising the second layer ranges from 0.7x10⁻³ and 10x10⁻³ kWh/m².

19. The process according to the preceding claim whose fibrous layers are formed from unbleached natural fibres and are hydroentangled before undergoing any chemical treatment of bleaching or boiling-off.

20. The process according to Claim 18 whose fibrous layers are formed from unbleached natural fibres and are bleached or boiled-off before being hydroentangled.

## Patentansprüche

1. Hautpflegeerzeugnis mit Peelingwirkung durch Reibung, das mindestens eine erste und eine zweite äußere Schicht aus einem Faserstoff umfasst, **dadurch gekennzeichnet, dass** die erste Schicht (10, 10") eine absorbierende Lage bildet und im Wesentlichen aus Zellulosefasern mit einem geringen Micronaire-Wert, insbesondere unter 8, besteht, und die zweite Schicht (20, 20', 20") im Wesentlichen aus Fasern mit einem hohen Micronaire-Wert, insbesondere mit einem Micronaire-Wert von über 8, besteht, die aus den folgenden Materialien ausgewählt sind: Leinen, Ramie, Sisal, Jute, Hanf, allein oder vermischt.

2. Erzeugnis nach dem vorhergehenden Anspruch, dessen zweite Schicht (20, 20', 20") mindestens 50 %, vorzugsweise 70 %, Fasern mit einem hohen Micronaire-Wert, und höchstens 50 %, vorzugsweise 30 %, weitere Naturfasern, Synthesefasern oder Chemiefasern umfasst.

3. Erzeugnis nach einem der Ansprüche 1 und 2, dessen erste Schicht mindestens 50 %, insbesondere mindestens 70 %, und genauer 100 %, der Zellulosefasern umfasst.

4. Erzeugnis nach Anspruch 3, dessen Zellulosefasern mit einem geringen Micronaire-Wert Baumwollfasern sind.

5. Erzeugnis nach einem der vorhergehenden Ansprüche, wobei die flächenbezogene Masse der ersten Schicht (10) zwischen 30 und 300 g/m² beträgt.

6. Erzeugnis nach Anspruch 3, dessen erste Schicht bis zu 50%, und insbesondere bis zu 30 %, Synthesefasern und/oder Chemiefasern umfasst.

7. Erzeugnis nach einem der Ansprüche 3 bis 6, dessen erste Schicht (10) ein Faservlies umfasst, das im Luftstromverfahren oder durch Kardieren hergestellt ist.

8. Erzeugnis nach einem der vorhergehenden Ansprüche, dessen erste Schicht (10) ein oder mehrere übereinanderliegende Krempelvliese umfasst.

9. Erzeugnis nach Anspruch 1 oder 2, wobei die flächenbezogene Masse der zweiten Schicht (20, 20', 20") zwischen 15 und 120 g/m² beträgt.

10. Erzeugnis nach einem der vorhergehenden Ansprüche, das eine dritte Schicht (20') umfasst, die im Wesentlichen aus Fasern mit einem hohen Micronaire-Wert besteht, wobei die erste Schicht (10) zwischen der zweiten und der dritten Schicht angeordnet ist.

11. Erzeugnis nach einem der vorhergehenden Ansprüche, wobei die Zellulosefasern der zweiten Schicht Leinenfasern sind.

12. Erzeugnis nach einem der vorhergehenden Ansprüche, dessen zweite Schicht (20, 20") und/oder gegebenenfalls dritte Schicht (20', 20") aus Krempelvlies gebildet sind.

13. Erzeugnis nach einem der vorhergehenden Ansprüche, dessen zweite Schicht oder gegebenenfalls dritte Schicht ein im Luftstromverfahren hergestelltes Vlies umfasst.

14. Erzeugnis nach einem der vorhergehenden Ansprüche, dessen Schichten miteinander verbunden sind.

15. Erzeugnis nach dem vorhergehenden Anspruch, dessen Schichten miteinander durch Wasserstrahlverfestigung verbunden sind.

16. Erzeugnis nach einem der Ansprüche 14 und 15, dessen Reißfestigkeit bei einer 25 mm breiten Probe zwischen 10 und 60 N beträgt.

17. Erzeugnis nach dem vorhergehenden Anspruch, das die Form eines Tuchs mit einer flächenbezogenen Masse zwischen 45 und 100 g/m² aufweist.

18. Verfahren zum Herstellen eines Erzeugnisses nach einem der Ansprüche 15 und 16, wobei die Wasserstrahlverfestigungsenergie, die auf die Seite, die die zweite Schicht umfasst, aufgebracht wird, zwischen 0,7x10⁻³kWh/m² und 10x10⁻³ kWh/m² beträgt.

19. Verfahren nach dem vorhergehenden Anspruch, wobei die Faserschichten aus natürlichen Rohfasern hergestellt und wasserstrahlverfestigt werden, bevor sie chemisch gebeucht und gebleicht werden.

20. Verfahren nach Anspruch 18, wobei die Faserschichten aus natürlichen Rohfasern hergestellt und gebeucht und gebleicht werden, bevor sie wasserstrahlverfestigt werden.
